# EUROPÄISCHE PATENTSCHRIFT

(11) **EP 1 027 379 B1**
(45) Veröffentlichungstag und Bekanntmachung des Hinweises auf die Patenterteilung: **28.02.2007**
(21) Anmeldenummer: 99946041.3
(22) Anmeldetag: 28.08.1999
(51) Int. Cl.: C08F 255/02, C07K 1/04, C08F 259/08, C08F 291/18, C08J 7/18

(54) **VERFAHREN ZUR HERSTELLUNG POLYMERER FESTPHASENTRÄGER**
METHOD FOR PRODUCING POLYMERIC SOLID PHASE SUPPORTING MATERIALS
PROCEDE DE FABRICATION DE SUPPORTS POLYMERES DE PHASE SOLIDE

(30) Priorität: 28.08.1998 DE 19840541
(43) Veröffentlichungstag der Anmeldung: 16.08.2000
(73) Patentinhaber: Poly-An Gesellschaft zur Herstellung von Polymeren für spezielle Anwendungen und Analytik mbH, 13086 Berlin (DE)
(72) Erfinder: ULBRICHT, Mathias, D-10407 Berlin (DE); VOLKMER-ENGERT, Rudolf, D-13349 Berlin (DE); GERMEROTH, Lothar, D-10717 Berlin (DE); WENSCHUH, Holger, D-12555 Berlin (DE)
(74) Vertreter: Schneider, Henry
(86) Internationale Anmeldenummer: PCT/EP1999/006294
(87) Internationale Veröffentlichungsnummer: WO 2000/012575

(56) Entgegenhaltungen:
- EP-A- 0 860 213
- EP-A- 0 872 512
- EP-A- 0 893 164
- EP-A- 0 893 165
- WO-A-92/04384
- WO-A-95/00533
- WO-A-95/09176
- ZHANG PEI YAO ET AL: "SURFACE MODIFICATION BY CONTINUOUS GRAFT COPOLYMERIZATION. \I. PHOTOINITIATED GRAFT COPOLYMERIZATION ONTO POLYETHYLENE TAPE FILM SURFACE" JOURNAL OF APPLIED POLYMER SCIENCE,US,JOHN WILEY AND SONS INC. NEW YORK, Bd. 40, Nr. 9 / 10, Seite 1647-1661 XP000162051 ISSN: 0021-8995

## Beschreibung

Die Erfindung betrifft ein Verfahren zur Herstellung von kontinuierlichen polymeren Festphasenträgern zur simultanen kombinatorischen Synthese von organischen Verbindungen mittels SPOT-Synthesetechnologie, bestehend aus einer polyolefinischen Trägermatrix und einzelnen Ketten eines Pfropfcopolymers, die durch heterogene photoinitiierte Pfropfcopolymerisation von Acrylat-. Vinyl- und Allylmonomeren auf der gesamten Oberfläche des Trägerpolymers synthetisiert werden und Hydroxyl-, Carboxyl-, Amino-. Mercapto-. Aldehyd-, oder Halogen-Reaktivgruppen enthalten, die zur weiteren Derivatisierung genutzt werden können.

### Beschreibung des Standes der Technik

Die chemische Synthese von Verbindungen unter Verwendung des Konzeptes kombinatorischer Bibliotheken hat einen bedeutenden Einfluß auf den Prozeß der Entwicklung potentieller Kandidaten für neue Therapeutika und Diagnostika. Kombinatorische Chemie ist eine Technik, bei der eine große Anzahl strukturell unterschiedlicher Verbindungen unter vergleichbaren Reaktionsbedingungen kosten- und zeiteffektiv hergestellt und nachfolgend Hochleistungs-Screeningassays zur biologischen Testung zugeführt werden kann. Dabei nimmt die Weiterentwicklung von Merrifields Festphasensynthesestrategie [Merrifield, R.B.: J. Am. Chem. Soc. 1963, 85, 2149], ursprünglich für die Synthese von Peptiden eingeführt, eine Schlüsselposition ein. Da die Verwendung überschüssiger Reagenzien nahezu vollständige Reaktionen möglich macht, die Methodik leicht zu automatisieren ist und biologische Testsysteme auch direkt auf den polymeren Oberfächen anwendbar sind, wird die kombinatorische Chemie bevorzugt an fester Phase durchgeführt.

Die Modifizierung und Weiterentwicklung der urprünglichen Polystyren- und später eingesetzten Polyamidträgersysteme [Atherton, E.: Clive. D.L.J.; Sheppard, R.C.; J. Am. Chem. Soc. 1975, 97, 6584] führten zu neuen Polymeren, wie z.B. Tentagel- und PEGA- Harzen [Rapp, W.; Zhang, L.; Häbich, R.; Bayer, E. In Peptides 1988, (G. Jung & E. Bayer, Eds.) Walter de Gruyter, Berlin, New York, S.199; Meldal, M.; Tetrahedron Lett. 1992, 33, 3077]. Diese wurden zunehmend auch zur Festphasensynthese nichtpeptidischer Substanzen eingesetzt. Obwohl spherische Harzkügelchen aus unterschiedlichen Materialien weit verbreitet sind, haben andere physikalische Erscheinungsformen wie Stäbchen (Polyethylen) oder kontinuierliche Oberflächen (Zellulose, Baumwolle, Glas, Polyolefine) zu neuen Synthesetechniken geführt.

Ein solches Synthesekonzept ist die Methode der Spatially Addressable Combinatorial Libraries [Review: Pirrung, M.C.; Chem. Rev. 1997, 97, 473]. Ein entscheidender Vorteil solcher Substanzbibliotheken besteht darin, daß die Position, an der sich ein synthetisiertes Molekül auf dem Polymer befindet, seine Zusammensetzung beschreibt. Somit kann bei Verwendung von in Format und Struktur geeigneten Trägermaterialien die Anwendung kompatibler biologischer Testsysteme direkt zu Informationen bezüglich der biologischen Aktivität der synthetisierten Substanzen führen.

Somit haben das Erscheinungsbild, die chemischen sowie physikalischen Eigenschaften und die Oberflächenfunktionalisierung der Trägermaterialien einen entscheidenden Einfluß sowohl auf die Qualität und Effizienz der Synthese als auch auf die Kompatibilität mit biologischen Testsystemen.

Die von Frank [Frank, R.; Tetrahedron, 1992, 48, 9217] entwickelte SPOT-Technologie zum Aufbau von Peptiden an planaren Zelluloseoberflächen ermöglicht dabei in besonderem Maße den effizienten parallelen Aufbau großer Zahlen von Peptidsequenzen. Die Methode, bei der die Reagenzien örtlich addressiert auf den kontinuierlichen Träger pipettiert werden, zeichnet sich desweiteren durch eine Anwendbarkeit von herkömmlichen Screeningassays aus, da die Auslesegeometrien der Hochleistungstestsysteme kompatibel mit den flächigen Trägermaterialien sind. So konnten beispielsweise SPOT-Arrays, neben dem klassischen Epitopmapping, eingesetzt werden, um optimale Bindungssequenzen von Proteinkinasen bzw. metallbindende Peptide zu entdecken [Tegge, W.; Frank, R.; Hofmann, F., Dostmann, W.R.G.; Biochemistry 1995, 34, 10569 ; Malin, R.; Steinbrecher, R.; Jannsen, J.; Semmler, W.; Noll, B.; Johannsen, B.; Frömmel, C.; Höhne W.: Schneider-Mergener, J.; J. Am. Chem. Soc. 1995, 117, 11821].

Aufgrund der limitierten chemischen und mechanischen Stabilität der Zellulosemembranen ist die Technologie bisher jedoch auf die Herstellung von Peptidsequenzen, die unter relativ milden Synthesebedingungen erfolgt, beschränkt.

Bedingt durch die Struktur des chemisch homogenen Trägers und die Nutzung von Hydroxygruppen als Reaktivgruppe führt der permanente Überschuß an Hydroxyfunktionalität auf der Zelluloseoberfläche dazu, daß selektive bzw. vollständige Umsetzungen nicht zu erreichen sind. Dies ist besonders für die Generierung unterschiedlicher Linkerkonstrukte ein Nachteil, was die Vielfalt möglicher Reaktionen entscheidend einschränkt.

Einige Entwicklungen auf dem Gebiet von flächigen Trägermaterialien könnten u.U. die Nachteile der Zellulose bei der SPOT-Synthese überwinden, sind jedoch bisher nicht in der SPOT-Synthesetechnik angewandt worden.

So beschreiben die Patente von Berg et al. (WO 90/02749, WO 91/13098) und Batsberg et al. (WO 95/00533) die Verwendung von Polyolefinfilmen (vorzugsweise Polyethylen). Diese wurden durch γ-Strahlen oder mittels chemisch initiierter Pfropf- und Homopolymerisation von Styren aus alkoholischen Lösungen modifiziert. Eine weitere chemische Derivatisierung zur Einführung von Reaktivgruppen folgte. Die so erzeugten Träger wurden in Reaktoren zur Peptid- und Oligonukleotidsynthese verwendet. Außerdem konnten Festphasenassays (ELISA) durchgeführt werden.

Obwohl die Pfropfung von Polystyrenketten eine logische Weiterentwicklung des klassischen Merrifield-Harzes zu einem flächigen Träger unter Beibehaltung der herkömmlichen Peptidsynthesebedingungen ist, ist die Auswahl dieses Polymers in diesem Kontext nicht zwingend. Sie bringt im Gegenteil sogar Nachteile, wie z.B. die stark unterschiedliche Solvatation der Ketten in unterschiedlichen Lösungsmitteln. Desweiteren ist Polyethylen als bevorzugtes Basispolymer mechanisch wenig stabil, und die beschriebenen Filme bzw. anderen Formkörper sind generell nicht porös. Die mögliche Funktionalisierung ist damit auf die äußere Oberfläche beschränkt (d.h. dicke oder dünne Pfropfschicht), was zu einer geringen Beladungskapazität führt. Eine oberflächenselektive Funktionalisierung ist nicht gewährleistet. Durch die Wahl von γ-Strahlung zur Initiierung und insbesondere des Monomer/Lösungsmittelsystems werden zwar zusätzlich auch Reaktionen (z.B. Pfropf- und Homopolymerisation) im Polyolefinfilm ausgelöst, diese verstärken jedoch lediglich die mechanische Instabilität und verursachen eine Trübung, die die Anwendung in Assays behindert.

Weiterhin wurden von Hudson et al. (WO 94/05394) neben der Verwendung von porösen Sinterplatten auch mit Aminogruppen funktionalisierte Polypropylenmembranen beschrieben, an die über einen Spacer (PEG) ein hydrophiles Polymer (Dextran, partiell hydrolysiertes Chitin) kovalent gekoppelt ist. Die in kleinen Reaktoren (Rasterplatten mit Löchern) verwendeten hydrophilen Träger besitzen eine einheitliche Reaktivität und eignen sich gut für Screeningzwecke. Allerdings ist die harsche Primärmodifizierung via Chromoxidation besonders mit Hinblick auf den Einsatz von porösen Trägern (mit größerer spezifischer Oberfläche und damit empfindlicher Morphologie) nachteilig. Deshalb lassen sich die Membranen offensichtlich nur aufgrund der Fixierung in der Rasterplatte, d.h. als diskontinuierlicher Träger, handhaben.

Desweiteren führt die relativ aufwendige Konstruktion der Synthesevorrichtung zu einer streng limitierten Kapazität und Variabilität der einzelnen Syntheseareale.

Das Patent von Köster et al. (EP 0305929) beschreibt die Synthese von Peptiden und Oligonukleotiden an porösen Membranpolymeren, an denen durch strahlungsinitiierte Radikalerzeugung Reaktivgruppen auf der Oberfläche erzeugt wurden. Die Funktionalisierung über (aufgrund der Auswahl von Initiator und Monomeren) unterschiedlich stark vernetzte Polymerschichten kann jedoch zu ungleichmäßigen Zugänglichkeiten der funktionellen Gruppen führen. Damit erscheinen diese Membranen für die SPOT-Synthese als unzweckmäßig. So ist die Eignung großer Flächen für Synthese und Bioassays (die Stabilität und gleiche Zugänglichkeit aller Reaktivgruppen erfordern) nicht erkennbar. Generell sind Komplikationen bei der Festphasensynthese von längeren oder/und komplizierteren Sequenzen zu erwarten.

Coassin et al. (WO 95/09176) berichten über eine Polymerfunktionalisierung (Polypropylen) mit geeignetem Reaktivplasma (z.B. Ammoniak), um zu Filmen bzw. Membranen als Träger für die Synthese bzw. Sequenzierung von Biomolekülen zu kommen. Die Funktionalisierung wird jedoch von Nebenreaktionen (Oxidation) begleitet, die die mechanische Stabilität des Trägers deutlich verschlechtern. Außerdem ist die Plasmafunktionalisierung bekanntermaßen wenig chemoselektiv. Das Resultat ist eine geringe Beladung mit Reaktivgruppen, da die Modifizierung nur oberflächlich erfolgt und die Flüsse von Ladungsträgern und reaktiven Spezies (bei reaktiven Plasmen) wegen der Degradationseffekte (s.o.) minimiert werden müssen.

Großflächige kontinuierliche Träger wurden auch von Eichler (DD 27285A1), Lebl et al. (EP 0385433 A2, EP 0445915 A1) und Okrongly et al. (EP 0400920) beschrieben. Während sich die beiden ersten Patente auf die Modifizierung an Zellulose mit den bekannten Nachteilen beziehen, werden im letzteren vorzugsweise Mikrotiterplatten (Polystyren) für die Peptidsynthese oberflächenfunktionalisiert. Einer vorteilhaften Kontrolle des Reaktionsablaufes mittels UV-Spektroskopie stehen hier jedoch die Nachteile einer begrenzten Bindungskapazität sowie einer eingeschränkten Beweglichkeit der Zielmoleküle, jeweils wegen der Beschränkung der Funktionalisierung auf eine einfache polymeranaloge Reaktion an der nichtporösen Polystyrenoberfläche, gegenüber.

Alle bislang bekannten Systeme (funktionelle Träger & Synthesebedingungen) haben aus Sicht der Anforderungen der SPOT-Synthese jeweils spezielle gravierende Nachteile, die eine effektive Anwendung verhindern. Insbesondere die Erweiterung der Anwendung der SPOT-Synthese für die Generierung von organisch-chemischen Substanzbibliotheken ist mit bekannten kontinuierlichen Festphasenträgern nicht möglich. Diese Nachteile bzw. Limitierungen werden mit der im folgenden beschriebenen Erfindung überwunden.

Die Aufgabe wird gelöst durch ein Verfahren zur Herstellung eines kontinuierlichen polymeren Festphasenträgers, umfassend eine Trägermatrix mindestens eines polymeren Materials sowie kovalent an die Trägermatrix gebundene Pfropfcopolymerketten, welche mit organischen Verbindungen reagible Reaktivgruppen aufweisen, wobei die Pfropfcopolymerketten räumlich definierte Reaktionsorte ausbilden, mit den Schritten
(a) Beschichten einer Oberfläche der Trägermatrix mit einem Photoinitiator, der geeignet ist, nach UV-Anregung Radikale durch Wasserstoffabstraktion an der Oberfläche der Trägermatrix zu erzeugen, wobei vor der Beschichtung keine Vorbehandlung der Oberfläche mit einem Photoinitiator und mindestens einem Monomer erfolgt,
(b) Belichten der beschichteten Trägermatrix mit UV-Strahlung in Gegenwart mindestens eines ungesättigten funktionellen Monomers, umfassend eine Reaktivgruppe als auch eine polymerisationsfähige Gruppe, wobei die über die polymerisationsfähige Gruppe kovalent an die Trägermatrix gebundenen Pfropfcopolymerketten entstehen, und wobei die Belichtung in Abwesenheit eines vernetzenden Monomers durchgeführt wird, und
(c) Extraktion nicht umgesetzter funktioneller Monomere, des Photoinitiators sowie löslicher Folgeprodukte.

### Beschreibung der Erfindung

Inhalt der vorliegenden Erfindung ist ein Verfahren zur Herstellung von neuartigen polymeren Oberflächen, an denen organische Substanzbibliotheken unter Verwendung des SPOT-Synthesekonzeptes hergestellt werden können, deren biologische Aktivität direkt auf dem Träger evaluiert und ausgelesen werden kann.

Erfindungsgemäß werden oberflächenfunktionalisierte Festphasenträger synthetisiert, die die in der Beschreibung des Standes der Technik aufgezeigten nachteiligen Eigenschaften vorhandener Träger überwinden bzw. durch neue Funktionalisierungsstrategien völlig alternative Synthesekonzepte und -techniken der kombinatorischen Synthese möglich machen.

Für die speziellen Anforderungen der SPOT-Synthese sind die guten mechanischen Eigenschaften (Stabilität) des Trägers und eine wohldefinierte Oberflächenfunktionalität gleichermaßen wesentlich. Allerdings gibt es keine homogenen Materialien, die beide Eigenschaften a priori miteinander vereinen. Das Beispiel der chemisch homogenen Zellulose, wo bei der Anwendung als fester, hydroxyl-reaktiver Träger immer wieder neue potentielle Hydroxyl-Reaktivgruppen aus der Kohlenhydratmatrix hervorkommen, lehrt vielmehr, daß ein optimaler Träger chemisch heterogen sein sollte. Eine Trägermatrix, die i.w. die Stabilität bedingt, mit einer chemisch verschiedenen funktionellen Oberfläche. Folglich stellt die heterogene Funktionalisierung von geeigneten polymeren Matrixmaterialien den attraktivsten Syntheseweg für neue, leistungsfähigere kontinuierliche polymere Festphasenträger dar. Wenn es gelingt, diese Funktionalisierung wirklich oberflächenselektiv zu führen, d.h. die chemische Umwandlung des Matrixpolymers auf dessen Oberfläche zu beschränken, dann können Trägermatrix und funktionelle Trägeroberfläche unabhängig voneinander optimiert werden.

Die Anforderungen an die Trägermatrix resultieren daraus, daß eine gute, sichere Handhabbarkeit vor, während und nach den Multischritt-Synthesen entscheidend für Erfolg bzw. Qualität von Synthesen und Assays sind. Andererseits ist eine große spezifische Oberfläche in Kombination mit geeigneter Porenstruktur der Trägermatrix die Voraussetzung für eine hohe Bindungskapazität pro Fläche. Eine hinreichende Dimensionsstabilität (minimale Quellung) muß aber auch gegeben sein, um eine Repositionierung der Membran während der Synthesen und definierte Produkte in jedem Spot zu erhalten und die Assayergebnisse unverfälscht auslesen zu können. Alle diese Anforderungen bedingen, daß die Trägermatrix -trotz gewünschter großer spezifischer Oberfläche- eine ausgezeichnete mechanische, thermische und Lösungsmittelbeständigkeit unter Synthese- und Assaybedingungen aufweisen muß.

Die Anforderungen an die funktionelle Oberfläche hinsichtlich der Synthese sind Reaktivgruppen in großer Menge (mindestens bis zu 1 µmol/cm²) und mit guter, konstanter Zugänglichkeit (über alle Synthesestufen für die Zielmoleküle). Das erfordert eine große spezifische Oberfläche in Kombination mit geeigneter Porenstruktur der Trägermatrix (s.o.) sowie Reaktivgruppen an Spacergruppen oder -ketten, eine gute Solvatation der Funktionalgruppen und eine optimale Benetzung der äußeren Trägeroberfläche (definierte Spots mit minimalen radialen Konzentrationsgradienten; keine Chromatographieeffekte). Dadurch werden eine hohe Produktreinheit (pro Spot) und identische Produktmengen für alle Spots erhalten.

Hinsichtlich der Assays sind weiterhin die Zugänglichkeit und Beweglichkeit der synthetisierten Moleküle sowie minimale unspezifische Wechselwirkungen der funktionellen Oberfläche mit den Testproteinen (minimaler Hintergrund beim Auslesen des Ergebnisses) wesentlich.

Das Herstellungsverfahren soll robust, reproduzierbar und flexibel in Bezug auf die Funktionalität und ggf. auch die Trägermatrix sein sowie ein problemloses Up-scaling auf große Flächen ermöglichen. Für das Syntheseprodukt, die neuen kontinuierlichen polymeren Festphasenträger, sind insbesondere die Homogenität und die Stabilität von Trägermatrix, funktioneller Schicht und deren Komposit unter Synthese- und Assaybedingungen kritisch.

### Synthese der neuen kontinuierlichen polymeren Festphasenträger

Ein als Trägermatrix besonders gut geeignetes Polymer ist Polypropylen, da es in weiten Bereichen von potentiellen organisch-chemischen Synthesebedingungen (einschließlich Lösungsmittel und Temperatur) mechanisch belastbar und chemisch stabil ist. Auch eine Quellung tritt, von sehr unpolaren Lösungsmitteln abgesehen, die aber für die Synthese keine Rolle spielen, kaum auf. Polypropylen ist sowohl als dünner, homogener, transparenter Film, als dicke Folie oder Platte, aber auch als poröse Membran mit unterschiedlichen Porenmorphologien und -größen verfügbar. Je nach Zielstellung (gewünschte Kapazität - spezifische Oberfläche; mechanische Flexibilität) können aus diesem Spektrum Basismaterialien für die Synthese von Festphasenträgern gewählt werden. Vorzugsweise werden makroporöse Flachmembranen aus Polypropylen (z. B. Mikrofiltrationsmembranen, hergestellt durch Phaseninversion und mit schwammartigem Porennetzwerk mit nominalen Porengrößen/Trenngrenzen von 0.2 µm, oder Filter, mit einer gesponnenen und gesinterten vliesartigen Netzwerkstruktur mit nominalen Porengrößen/Trenngrenzen von 0.6 µm) als Trägermatrix gewählt. Selbstverständlich können auch andere Polymere, wie z.B. Polyethylen, Polyvinylidenfluorid, vernetztes Polystyren oder Teflon, mit unterschiedlichen Morphologien eingesetzt werden. Die Trägermatrix kann auch extern bzw. intern durch einen zusätzlichen inerten Träger bzw. Partikel, Fasern oder Netzwerke aus Polymer, Glas oder Metall verstärkt werden.

Um eine maximale Stabilität unter Anwendungsbedingungen zu gewährleisten, wird ein Komposit aus der Trägermatrix und einer daran chemisch verankerten funktionellen Schicht synthetisiert. Die Trägermatrix wird durch photoinitiierte heterogene Pfropfcopolymerisation (streng oberflächenselektiv bezüglich der Polypropylenmatrix, aber über die gesamte Schichtdicke nahezu gleichmäßig) mit langen Pfropfpolymerketten aus funktionellen Monomeren kovalent funktionalisiert. Die erfindungsgemäßen neuen kontinuierlichen polymeren Festphasenträger für die SPOT-Synthese werden dabei nach einem Verfahren erhalten, das aus folgenden wesentlichen Schritten besteht:
i) Oberflächenbeschichtung der Trägermatrix mit einem Photoinitiator der nach UV-Anregung (durch Wasserstoffabstraktion und damit ohne Matrixpolymerdegradation) an der Trägeroberfläche Radikale erzeugen kann,
ii) UV-Belichtung der beschichteten Trägermatrix in Gegenwart eines funktionellen Monomeren oder Monomerengemisches unter Bildung einer funktionellen Schicht, die aus am Matrixpolymer kovalent verankerten, unvernetzten funktionellen Pfropfcopolymerketten besteht (die UV-Belichtung erfolgt vorzugsweise selektiv, so daß nur der Photoinitiator angeregt wird),
iii) Extraktion von nicht umgesetztem Monomer und Photoinitiator sowie von löslichen Homo- oder Copolymeren bzw. Photoinitiatorfolgeprodukten.

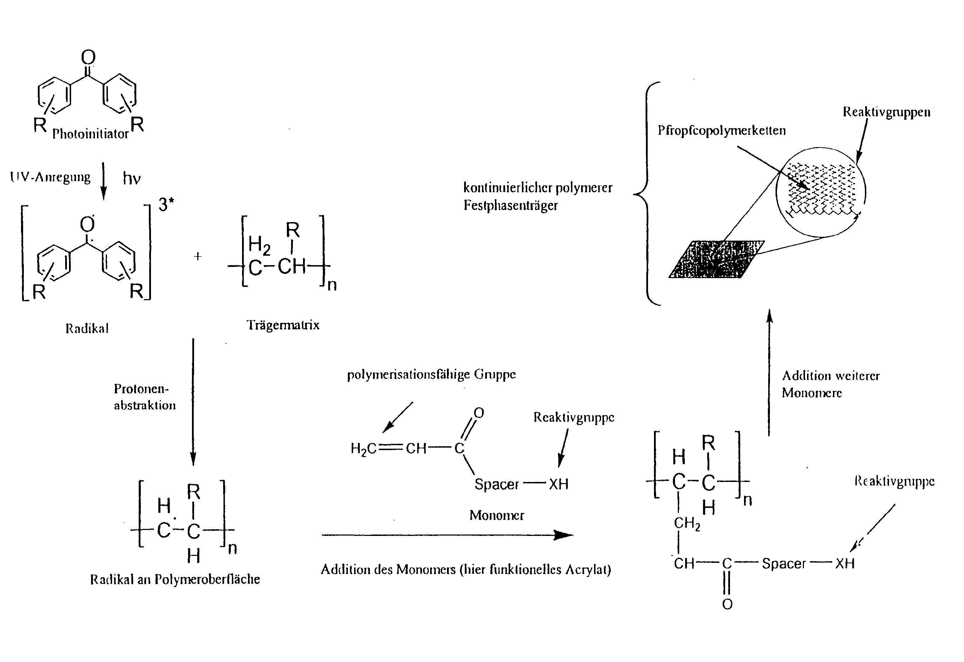

Abbildung 1 stellt schematisch das Herstellungsverfahren für den kontinuierlichen planaren Festphasenträger dar.

Auch eine sequentielle Aktivierung/Initiierung der Pfropfcopolymerisation ist möglich, indem zunächst die Belichtung der nach i) mit Photoinitiator beschichteten Trägermatrix in Gegenwart von Sauerstoff oder mit nachträglicher Exposition in Sauerstoff unter Bildung von Trägerpolymer-Peroxiden erfolgt und danach die Reaktion mit dem Monomer thermisch initiiert wird. Auch andere heterogen chemisch initiierte Reaktionen zur Initiierung einer Pfropfcopolymerisation sind anwendbar.

Als Photoinitiator eignen sich besonders Benzophenon und strukturell verwandte Ketonderivate. Die Beschichtung mit dem Photoinitiator in Stufe i) kann aus einer Lösung in einem Nichtlösurtgsmittel für das Trägerpolymer durch Dip-Coating oder Imprägnieren vorgenommen werden; sie kann aber gegebenenfalls auch ohne zusätzlichen Verfahrensschritt direkt vor der in Stufe ii) beschriebenen Pfropfcopolymerisation erfolgen, indem der Photoinitiator aus einer Mischung aus Initiator, Monomer bzw. Monomergemisch und ggf. Lösungsmittel an der Trägeroberfläche adsorbiert wird.

Den verwendeten Monomeren ist ein struktureller Aufbau gemeinsam, der in modualer Weise nach einem einheitlichen Reaktionsmechanismus (heterogene Pfropfcopolymerisation) zu einer Vielzahl von Oberflächenfunktionalitäten fuhren kann:
Reaktivgruppe - Spacergruppe - polymerisationsfähige Gruppe

Vorzugsweise werden funktionelle Acrylate, Methacrylate, Acrylamide oder Methacrylamide eingesetzt, aber auch andere funktionelle Vinylmonomere u.v.a.m. sind prinzipiell geeignet. Beispiele für Reaktivgruppen als Anker für die Festphasensynthese sind Hydroxyl- oder Aminogruppen. Durch funktionelle Monomere eingeführte Carboxyl- oder Epoxygruppen können auch sehr leicht (z.B. nach "Umfunktionalisierung" zu Aminogruppen) dafür genutzt werden. Als Spacergruppen sind Oligo- oder Polyethylenglykolketten besonders bevorzugt, da sie in vielen verschiedenen Lösungsmitteln ähnlich gut solvatisiert werden, eine gute Beweglichkeit garantieren und auch die Biokompatibilität verbessern. Spaltbare Linkerstrukturen können z.B. Ester (in Acrylaten) oder Amide (in Acrylamiden) sein, wobei letztere eine wesentlich bessere Hydrolysestabilität aufweisen und für Festphasensynthesen unter harschen organisch-chemischen Bedingungen zu bevorzugen sind. Hydroxy- oder Amino(polyethylenglycol)methacrylate bzw. -methacrylamide sind Beispiele für Monomere, nach deren Pfropfcopolymerisation Träger resultieren, die direkt mit besonderen Vorzügen für die SPOT-Synthese verwendet werden können. Mit Pfropfpolyacrylsäure funktionalisierte Träger lassen sich einfach mit Diamino-oligoethylenelycolen zu hydrolysestabilen Amino-Trägem "umfunktionalisieren".

Zur Präparation von Pfropfketten aus Copolymerisaten können o.g. funktionelle Monomere in Mischung mit anderen funktionellen, aber auch mit inerten Monomeren eingesetzt werden. Letzte können zur "Verdünnung" reaktiver Gruppen am/im Festphasenträger oder zur Einstellung der Hydrophilie/phobie bzw. Ladung (u.U. auch wesentlich für die Assaykompatibilität) genutzt werden.

Bei vorgegebener Trägermatrix, mit entsprechender homogener oder poröser Struktur und damit geringer oder größerer spezifischer Oberfläche, lassen sich über die Pfropfcopolymerisationsbedingungen (Photoinitiatorbeschichtung, Monomerkonzentration, Belichturtgsdauer) der Funktionalisierungsgrad und damit die Beladung mit Reaktivgruppen pro Fläche in weiten Bereichen (bis zu 10 µmol/cm²) einstellen. Durch die Pfropfcopolymerketten wird in jedem Fall eine Kapazitätsvergrößerung verglichen mit einer polymeranalog funktionalisierten glatten Trägeroberfläche erzielt. Bei Wahl geeigneter Bedingungen (Lichtabsorption von Photoinitiator und Trägermatrix) sind gleichmäßige Funktionalisterungen dicker poröser Schichten (mit BP bei PP-Membranen bis zu 200 µm) möglich.

Die vorzugsweise angewandte Photofunktionalisierung gestattet in einem schnellen und effektiven Verfahren die reproduzierbare und gleichmäßige Funktionalisierung großer kontinuierlicher Festphasenträgerflächen.

### Synthesen auf den neuen kontinuierlichen polymeren Festphasenträgern

Durch das gewählte Herstellungsverfahren wird es möglich, auf einer festen Trägermatrix primäre Reaktivgruppen, vorzugsweise Amino- bzw. Hydroxylgruppen, in großer Anzahl (> µmol/ cm²) bei guter Zugänglichkeit zu generieren. Interessanterweise bleibt diese Zugänglichkeit auch über eine größere Zahl von Synthesezyklen (10% Beladungsabfall nach ca. 10 Synthesezyklen) nahezu konstant. Die Ursache dafür ist in einer großen spezifischen Oberfläche in Kombination mit geeigneter Porenstruktur der Trägermatrix des Trägers (poröse Membran) sowie einer sehr guten Beweglichkeit der Reaktivgruppen, bedingt durch solvatisierte Pfropfcopolymerketten und weitere Spacergruppen, zu finden. Desweiteren führt die optimale Benetzung der äußeren Trägeroberfläche zu definierten Spots mit minimalen Tiefenfiltrationseffekten und radialen Konzentrationsgradienten. Wegen der dadurch konstanten Reagenzienüberschüsse an jedem Ort der Benetzung kann eine nahezu konstante Produktqualität gewährleistet werden. Aufgrund der Homogenität des Festphasenträgers (identische Anzahl von gleich zugänglichen Funktionalitäten über die gesamte Fläche) können gleiche Produktmengen pro Spot generiert werden. Darüberhinaus führt die verringerte Hydrophobie der modifizierten Festphasenträger im Vergleich zur Trägermatrix in Verbindung mit der durch die Spacerstrukturen induzierten Beweglichkeit der gebundenen Substanzen zu einer guten Assaykompatibilität.

Die polymeren Festphasenträger zeichnen sich weiterhin durch eine hohe chemische Stabilität, das heißt Resistenz gegenüber starken Säuren. Laugen, Lösungsmitteln sowie oxidierenden und reduzierenden Reagenzien aus. Desweiteren besitzen die neuen polymeren Festphasenträger eine hohe Beständigkeit gegenüber physikalischen Einflüssen wie Temperatur, Ultraschall- und Mikrowellenbehandlung, was dazu führt, daß die kontinuierlichen Oberflächen auch nach mehreren Reaktionszyklen keine oder nur vernachlässigbare Quellung zeigen. Diese Eigenschaften lassen eine wiederholte, örtlich adressierte Pipettierung kleinster Volumina (10 nl) und im Gegensatz zur Zellulose eine weitgehende Variation der chemischen Reaktionsbedingungen zu.

Die neuen kontinuierlichen polymeren Festphasenträger können mit einer Vielzahl von Linkerkonstrukten zur Aufnahme von weiteren Molekülen vorbereitet werden. So sind neben säuresensitiven Linkern (z B. Rink-Linker), UV-aktivierbare Photolinker (z. B. 4-(Methoxy-4-(2-Fmoc-amino-ethyl)-5-nitrophenoxylbutansäure) auch basenlabile Linker (z. B. HMBA-Linker) oder mit wässrigen Puffersystemen spaltbare Linkerkonstrukte (z. B. Imidazollinker) einsetzbar. Diese Linker sind auf dem kontinuierlichen Festphasenträger durch örtlich definierte Applikation von Spots auch parallel anwendbar und können z.B. zur Synthese von multipel oder graduell abspaltbaren Produkten genutzt werden.

Obwohl auch peptidische Substanzen mit gleicher oder besserer Qualität als bei der herkömmlich verwendeten Zellulose hergestellt werden können, ergeben sich aus den oben genannten Eigenschaften der neuen Festphasenträger vor allem vielfältige Möglichkeiten für die Synthese von organisch-kombinatorisch hergestellten Substanzbibliotheken. Da die neuen Festphasenträger erfindungsgemäß eine hohe chemische und physikalische Stabilität besitzen, liegen ihre entscheidenden Vorteile besonders in der kombinatorischen Synthese von Bibliotheken aus nichtnatürlichen Oligomeren (z. B. Peptoiden, Oligocarbamaten, Oligoharnstoffen, Azatiden, Ketiden, Peptid-Sulfonamiden, vinylogen Sulfonylpeptiden) sowie kleinen synthetischen Molekülen (z. B. Benzodiazepinen, Triazinen, Triazolen, Hydantoinen, Cubanen, Xanthinen, Pyrollidinen, β-Laktamen, Thiazolidonen). Weiterhin liegt ein Anwendungsschwerpunkt in der Parallelsynthese von Chimären (Konjugate) aus unterschiedlichen synthetischen (natürlich oder unnatürlichen) Oligomeren bzw. kleinen synthetischen Molekülen mit Naturstoffen wie z. B. Steroiden oder Zuckermolekülen.

Vorteilhaft ist die Verwendung der kontinuierlichen Festphasenträger, welche nach dem erfindungsgemäßen Verfahren hergestellt worden, für die parallele und kombinatorische Synthese von trägergebundenen oder freien Verbindungen, die durch SPOT-Synthese von aktivierten Synthesebausteinen an verschiedenenen Reaktionsorten auf dem kontinuierlichen Festphasenträger synthetisiert werden.

Bevorzugt ist eine erfindungsgemäße Verwendung der kontinuierlichen Festphasenträger für die parallele und kombinatorische Synthese von Proteinen.

Mehr bevorzugt ist eine erfindungsgemäße Verwendung der kontinuierlichen Festphasenträger für die Festphasensynthese, vorzugsweise SPOT-Synthese, auch als Array von identisch oder unterschiedlich funktionalisierten Trägern bzw. als Stapel von identisch oder unterschiedlich funktionalisierten Trägern für die Synthese von multipel oder graduell abspaltbaren Verbindungen, wobei durch Split (Single Sheets) & Combine (Stapel) Techniken mit ganzen Trägern auch eine quasi dreidimensionale Kombinatorik möglich wird.

Noch mehr bevorzugt ist die erfindungsgemäße Verwendung der kontinuierlichen Festphasenträger für die Identifizierung von Molekülen (Liganden) an synthetisierten Verbindungen, umfassend die folgenden Schritte:
Inkubation mit dem Liganden, Entfernen von überschüssigen Liganden durch Waschen, Detektion gebundener Liganden durch geeignete Verfahren wie: i) immunologische Nachweise, ii) Nachweis gebundener radioaktiv markierter Liganden, iii) Fluoreszenz- oder Chemolumineszenz-Nachweise, iv) biosensorische Nachweise.

Am meisten bevorzugt ist die erfindungsgemäße Verwendung der kontinuierlichen Festphasenträger zur Untersuchung enzymatischer Aktivitäten an synthetisierten Verbindungen, umfassend die folgenden Schritte:
i.) Inkubation mit dem Enzym, ii.) Detektion enzymatischer Aktivität.

Die Erfindung wird im folgenden anhand von Beispielen näher beschrieben, welche die Synthese der neuen kontinuierlichen polymeren Festphasenträger, deren Anwendung in der SPOT-Synthese von Peptiden und anderen organisch-chemischen Molekülen sowie von Substanzbibliotheken aus Peptiden und anderen organisch-chemischen Molekülen, und die Anwendung in Analyse- und Screeningsystemen detaillierter erläutern.

### Definitionen

**Festphasensynthese:**
   kovalente Verknüpfung von Molekülen mit einem unlöslichem polymeren Täger und nachfolgende Reaktionen an den trägergebundenen Substanzen, wobei durch einfache Wasch- und Filtrieroperationen überschüssige Reagenzien einsetzbar und abtrennbar werden und das Zielprodukt bis zur Abspaltung am polymeren Träger gebunden bleibt
**Funktionelle Monomere:**
   Moleküle, die eine polymerisationsfähige Gruppe, eine Spacergruppe und eine Reaktivgruppe enthalten und unter dem Einfluß eines Polymerisationsinitiators lineare und/oder verzweigte Polymerketten bilden können
**Kontinuierlicher Festphasenträger:**
   flächiges Material aus einer Trägermatrix (s.u.) mit daran kovalent verankerten Pfropfcopolymerketten (s.u.)
**Ligand**
   Molekül, das an die auf dem kontinuierlichen Festphasenträger synthetisierten Verbindungen bindet und/oder sie chemisch umsetzt und das ein Protein, Enzym, Kohlenhydrat oder Glykoprotein, Lipide oder Lipoprotein, eine Nukleinsäure oder eine niedermolekulare Verbindung sein kann
**Linker:**
   molekulares spaltbares Ankermolekül, welches die Abspaltung der synthetischen Zielprodukte vom Festphasenträger erlaubt
**Pfropfcopolymerketten:**
   lineare und/oder verzweigte Molekülketten, aufgebaut aus funktionellen Monomeren (s.u.)
**Photoinitiator:**
   erzeugt nach Beschichten der Oberfläche eines Trägerpolymers und Anregung mittels UV-Strahlung durch Wasserstoffabstraktion vom Trägerpolymer Radikale, die mit einem funktionellen Monomer reagieren können, z.B. ein Benzophenonderivat oder Keton
**Polymerisationsfähige Gruppe:**
   reaktionsfähige Mehrfachbindung in funktionellen Monomeren, z.B. eine Acrylat-, Vinyl-, oder Allylgruppe
**Radikale:**
   elektrisch neutrale Moleküle, die ein magnetisches Moment besitzen, eine bevorzugte Reaktivität ist die Addition an Verbindungen, die reaktionsfähige Mehrfachbindungen (polymerisationsfähige Gruppen) enthalten
**Reaktivgruppe:**
   reaktionsfähige chemische Gruppe (Funktionalität) für kovalente Kupplungsreaktionen mit anderen Verbindungen, z.B. eine Hydroxylgruppe, Carboxylgruppe, Aminogruppe, Mercaptogruppe oder eine Halogengruppe
**Spacergruppe:**
   molekularer Abstandshalter zwischen polymerisationsfähiger Gruppe und Reaktivgruppe in einem funktionellen Monomer, z.B. ein Oligo- oder Polyethylenglykol oder eine Bindung
**SPOT**
   entsteht durch das Pipettieren von Reagentien auf zusammenhängende kontinuierliche
   Oberflächen, wobei das pipettierte Volumen die Größe des SPOTs und die Zahl der möglichen SPOTs pro Fläche definiert
**Spotten**
   das Pipettieren von Reagentien auf zusammenhängende kontinuierliche Oberflächen
**SPOT-Synthese:**
   Festphasensynthesekonzept bei dem durch Pipettierung von kleinen Reagenströpfchen auf ein vordefiniertes Array von Reaktionsorten auf einer zusammenhängenden kontinuierlichen Festphasenträger, die als polymerer Festphasenträger fungiert, SPOTs definiert werden: diese SPOTs stellen Mikroreaktoren dar, in denen Festphasensynthesen ablaufen können, wenn Lösungsmittel mit geringem Dampfdruck verwendet werden
**Trägermatrix:**
   chemisch und morphologisch stabiles, flächiges Material, das aus polyolefinischen Polymeren(Trägerpolymer) wie Polyethylen, Polystyren, Polyvinylidenfluorid, Polytetrafluorethylen, bevorzugt aber Polypropylen besteht
**UV-Anregung:**
   überführt den Photoinitiator in einen angeregten Zustand, ohne das Trägerpolymer anzuregen (zu degradieren)

### Ausführungsbeispiele

### Beispiel 1: Synthese von kontinuierlichen polymeren Festphasenträgern - Hydroxy-PEG-Ester-PP-Membranen mit unterschiedlichen Spacerlängen - unter Laborbedingungen

Eine PP-Membran (Accurel 2E, Akzo; nom. Porendurchmesser dₚ = 0.2 µm, Schichtdicke 150 µm; d = 80 mm) wird unter Schütteln für 2 h mit einer 100 mM Lösung von Benzophenon (BP) in Methanol equilibriert. Die Membran wird aus der Lösung entnommen und die Oberfläche schnell an der Luft getrocknet, ohne daß das Innere der Poren entnetzt wird. Danach wird die Membran in einer Petrischale (d = 90 mm) auf ein mit einem Teil der Monomerlösung (ca. 5 ml) getränktes Filterpapier gelegt, mit einem Glasring (d = 80 mm) fixiert und sofort mit dem Rest der Polymerisationslösung überschichtet (insgesamt 20 ml 50 g/l PEGMA 306 bzw. 78.5 g/l PEGMA 526, jeweils in mit BP gesättigtem Wasser; die Monomere -PEG-methacrylate, wobei die Zahl das mittlere Molekulargewicht beschreibt- sind Produkte der Polysciences Europe GmbH, Eppelheim). Die Petrischale wird in einem Reaktor plaziert, der danach mit einem UV-Filterglas-Deckel (Cut-off 310 nm) verschlossen und für mindestens 30 min mit Stickstoff gespült wird. Anschließend erfolgt - weiterhin unter Stickstoffspülung - für 30 min die UV-Belichtung (HBO 350 DeepUV; Hamamatsu, in einer Belichtungsapparatur von Oriel, 3 mW/cm²). Nach weiteren 15 min. unter Stickstoff wird die Membran entnommen und anschließend vollständig mit Wasser, Methanol und Aceton extrahiert. Die Bestimmung des Modifizierungsgrades (DG) der Membran erfolgt gravimetrisch: DG (PEGMA 306) = 1.12 mg/cm² =3.5 µmol/cm²; DG (PEGMA 526) = 1.37 mg/cm² = 2.6 µmol/cm².
Eine weitere Charakterisierung erfolgt durch FTIR-ATR-Spektroskopie, REM sowie Messung der Membranpermeabilität.

### Beispiel 2: Synthese von kontinuierlichen polymeren Festphasenträgern - Carboxyl-PP- bzw. Carboxyl-Polyethylen(PE)-Membranen - unter Laborbedingungen

Eine PP- (Accurel 2E, Akzo; dₚ= 0.2 µm, Schichtdicke 150 µm) bzw. PE-Membran (celgard 2500. Hoechst-Celanese dₚ = 0.2 µm, Schichtdicke 25 µm) mit einem Durchmesser von d = 80 mm wird unter Schütteln für 2 h mit einer 100 mM Lösung von BP in Methanol equilibriert. Die Membran wird aus der Lösung entnommen und die Oberfläche schnell an der Luft getrocknet, ohne daß das Innere der Poren entnetzt wird. Danach wird die Membran in einer Petrischale (d = 90 mm) auf ein mit einem Teil der Monomerlösung (ca. 5 ml) getränktes Filterpapier gelegt, mit einem Glasring (d = 80 mm) fixiert und sofort mit dem Rest der Monomerlösung überschichtet (insgesamt 20 ml 10 g/l Acrylsäure, AA. in mit BP gesättigtem Wasser). Die Petrischale wird in einem Reaktor plaziert, der danach mit einem UV-Filterglas-Deckel (Cut-off 310 nm) verschlossen und für mindestens 30 min mit Stickstoff gespült wird. Anschließend erfolgt - weiterhin unter Stickstoffspülung - für 30 min (PP) bzw. 15 min (PE) die UV-Belichtung (HBO 350 DeepUV; 3 mW/cm²). Nach weiteren 15 min. unter Stickstoff wird die Membran entnommen und anschließend vollständig mit Wasser und Aceton extrahiert. Die Bestimmung des Modifizierungsgrades (DG) der Membran erfolgt gravimetrisch:
DG(PP) = 220 µg/cm² = 3.1 µmol/cm²;
DG(PE) = 245 µg/cm² = 3.4 µmol/cm².
Eine weitere Charakterisierung erfolgt durch FTIR-ATR-Spektroskopie. REM, Farbstoffbindung (Toluidinblau) sowie Messung der Membranpermeabilität.

### Beispiel 3: Synthese von kontinuierlichen polymeren Festphasenträgern - Hydroxy-Amid-PP-Membranen - unter Laborbedingungen

Eine PP-Membran (Accurel 2E, Akzo; dₚ = 0.2 µm) mit einem Durchmesser von d = 80 mm wird unter Schütteln für 2 h mit einer 100 mM Lösung von BP in Aceton equilibriert. Die Membran wird aus der Lösung entnommen und die Oberfläche schnell an der Luft getrocknet, ohne daß das Innere der Poren entnetzt wird. Danach wird die Membran in einer Petrischale (d = 90 mm) auf ein mit einem Teil der Polymerisations-lösung (ca. 5 ml) getränktes Filterpapier gelegt, mit einem Glasring (d = 80 mm) fixiert und sofort mit dem Rest der Polymerisationslösung überschichtet (insgesamt 20 ml 40 g/l 2-Hydroxypropylmethacrylamid, 2HPMAm, in mit BP gesättigtem Wasser; Monomer von Polysciences). Die Petrischale wird in einem Reaktor plaziert, der danach mit einem UV-Filterglas-Deckel (Cut-off 310 nm) verschlossen und für mindestens 30 min mit Stickstoff gespült wird. Anschließend erfolgt - weiterhin unter Stickstoffspülung - für 45 min die UV-Belichtung (HBO 350 DeepUV; 3 mW/cm²). Nach weiteren 15 min. unter Stickstoff wird die Membran entnommen und anschließend vollständig mit Wasser und Aceton extrahiert. Die Bestimmung des Modifizierungsgrades (DG) der Membran erfolgt gravimetrisch: DG = 355 µg/cm² = 2.5 µmol/cm².
Eine weitere Charakterisierung erfolgt durch FTIR-ATR-Spektroskopie, REM, Farbstoffbindung (Toluidinblau) sowie Messung der Membranpermeabilität.

### Beispiel 4: Synthese von kontinuierlichen polymeren Festphasenträgern - Benzylchlorid-PP-Membranen - unter Laborbedingungen

Eine PP-Membran (AN06, Millipore Corp: dₚ = 0.6 µm) mit einem Durchmesser von d = 80 mm wird gewogen und anschließend unter Schütteln 2 h mit einer Lösung von BP in Aceton (150 mM) equlibriert. Die BP beschichtete, noch feuchte Membran wird in einer Petrischale (d = 90 mm) auf ein mit einem Teil der Polymerisationslösung getränktes Filterpapier gelegt, mit einem Glasring (d = 80 mm) fixiert und sofort mit dem Rest der Polymerisationslösung überschichtet (insgesamt 20 ml 20 g/l Vinylbenzylchlorid VBC, in mit BP gesättigtem Wasser unter Zugabe von 7 g/l Emulgator Bis[2-ethylhexyl]sulfosuccinat-Natriumsalz bei 10000 upm für 30 Sekunden mit einen Thurrax emulgiert).
Die Petrischale wird für 30 Minuten geschüttelt und mit einer Glasplatte (Tief-UV-Filter, Cut-off 310 nm) abgedeckt. Die Belichtung erfolgt bei Halblast an einem UV-Trockner (Beltron-GmbH; 1 min UV-Belichtung pro Passage durch die Belichtungszone). Anschließend wird die Membran 1 h in einem Soxhlet mit Methanol und 1 h bei 50°C in Wasser extrahiert. Danach wird die modifizierte Membran getrocknet und gravimetrisch der Modifizierungsgrad (DG) bestimmt.

DG = 1,5 mg/cm² = 9.S µmol/cm². Eine weitere Charakterisierung erfolgt durch FTIR-ATR-Spektroskopie. REM. Farbstoffbindung (Toluidinblau) sowie Messung der Membranpermeabilität.

### Beispiel 5: Synthese von - kontinuierlichen polymeren Festphasenträgern Hydroxy-PEG-Ester-PP-Membranen - unter Technologiebedingungen

Eine PP-Membran (Accurel 2E, Akzo; dₚ = 0.2 µm. bzw. AN06, Millipore Corp; dₚ = 0.6 µm) mit einer Fläche von 18 cm x 26 cm wird gewogen und anschließend in Lösung von BP in Methanol oder Aceton für 2 h bei Raumtemperatur geschüttelt. Die BP beschichtete, noch feuchte Membran wird in eine Schale mit 300 ml Monomerlösung (PEGMA 526 in mit BP gesättigtem Wasser) auf Filterpapier gelegt und getränkt. Die Schale wird mit einer Glasplatte (Tief-UV-Filter, Cut-off 310nm) abgedeckt. Die Belichtung erfolgt bei Halblast an einem UV-Trockner (Beltron GmbH; 1 min. UV-Belichtung pro Passage durch die Belichtungszone). Anschließend wird die Membran 1 h in einem Soxhlet mit Methanol und 1 h bei 50 °C in Wasser extrahiert. Danach wird die modifizierte Membran getrocknet und gravimetrisch der Modifizierungsgrad bestimmt. Die Charakterisierung der Membran erfolgt analog wie in Beispiel 1 beschrieben.
Mit der Wahl der Synthesebedingungen (Photomitiatorbeschichtung. Monomerkonzentration, Belichtungsdauer) kann der Modifizierungsgrad variiert und für unterschiedliche Trägermembrantypen eingestellt werden (Beispiele s. Tab. 1).

### Beispiel 6: Synthese von kontinuierlichen polymeren Festphasenträgern - Carboxyl-PP-Membranen - unter Technologiebedingungen

Eine PP-Membran (Accurel 2E, Akzo; dₚ = 0.2 µm, bzw. AN06, Millipore Corp; dₚ = 0.6 µm) mit einer Fläche von 18 cm x 26 cm wird gewogen und anschließend in Lösung von BP in Methanol oder Aceton für 5 min bei Raumtemperatur geschüttelt. Die BP beschichtete, noch feuchte Membran wird in eine Schale mit 300 ml Monomerlösung (AA in mit BP-gesättigtem Wasser) auf Filterpapier gelegt und getränkt. Die Schale wird mit einer Glasplatte (Tief-UV-Filter, Cut-off 310nm) abgedeckt. Die Belichtung erfolgt bei Halblast an einem UV-Trockner (Beltron GmbH; 1 min. UV-Belichtung pro Passage durch die Belichtungszone). Anschließend wird die Membran 1 h in einem Soxhlet mit Methanol und 1 bei 50 °C in Wasser extrahiert. Danach wird die modifizierte Membran getrocknet und gravimetrisch der Modifizierungsgrad bestimmt. Die Charakterisierung der Membran erfolgt analog wie in Beispiel 2 beschrieben.
Mit der Wahl der Synthesebedingungen (Photoinitiatorbeschichtung, Monomer-konzentration, Belichturtgsdauer) kann der Modifizierungsgrad variiert und für unterschiedliche Trägermembrantypen eingestellt werden (Beispiele s. Tab. 1).

**Tabelle 1: Technologie-Synthesebedingungen für ausgewählte Hydroxy-PEG-Ester- und Carboxyl-PP-Membranen (Membran: 1 0.2 µm, Accurel 2E; 2 0.6 µm, AN06; UV-Belichtung unter Halblast mit UV-Trockner, Beltron GmbH)**

| Membran | Photoinitiator-beschichtung | Monomer | Monomer (g/l) | Belichtung (min) | DG (mg/cm²) | DG (µmol/cm²) |
|---|---|---|---|---|---|---|
| 1 | 150mM BP/Aceton | PEGMA 526 | 78.5 | 15 | 1.28 | 2.4 |
| 1 | 150mM BP/Aceton | AA | 23.3 | 10 | 0.48 | 6.8 |
| 1 | 150mM BP/Aceton | AA | 50.0 | 10 | 0.64 | 9.0 |
| 2 | 100mM BP/Aceton | PEGMA 526 | 78.5 | 10 | 0.19 | 0.36 |
| 2 | 100mM BP/Aceton | PEGMA 526 | 100.0 | 10 | 0.34 | 0.65 |
| 2 | 100mM BP/Aceton | AA | 50.0 | 10 | 0.13 | 1.8 |
| 2 | 150mM BP/Aceton | AA | 50.0 | 5/5* | 0.17 | 2.4 |

| | | | | | | |
|---|---|---|---|---|---|---|
| * Membran beidseitig belichtet | | | | | | |

### Beispiel 7: Synthese von kontinuierlichen polymeren Festphasenträgern - chemische Derivatisierung einer Carboxyl-PP-Membran (Aminofunktionalisierung)

Ein Membranstück (0.2 µm; aus Beispiel 6) der Größe 18 cm x 26 cm wurde in einer Instrumentenschale 1 h mit 80 ml einer 2 M Lösung von Thionylchlorid in DCM behandelt. Die Lösung wurde abdekantiert und die noch feuchte Membran sofort mit 100 ml einer 30proz. Lösung von 4,7.10-Trioxa-1,13-tridecandiamin in DCM (v/v) versetzt. Nach 90 min. wurde abdekantiert und die Membran gewaschen (1 x DCM, 5 x Methanol, je 5 min.) und getrocknet (Schema I).

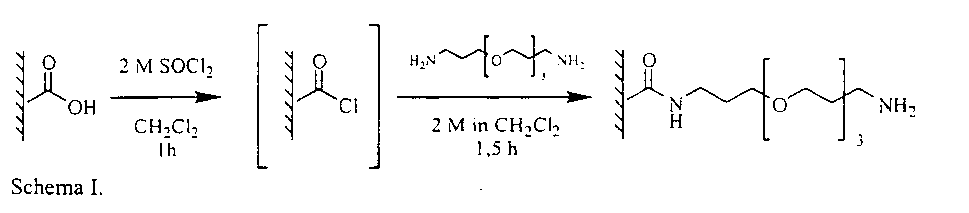

### Beispiel 8: Synthese von kontinuierlichen polymeren Festphasenträgern - chemische Derivatisierung einer Benzylchlorid-PP-Membran (Aminofunktionalisierung)

Ein Membranstück (0.2 µm; aus Beispiel 4) der Größe 5 cm x 3 cm wurde in einer Instrumentenschale 30 min mit einer 2,5 M Lösung von 4,7,10-Trioxa-1,13-tridecandiamin in DCM (v/v) bei Raumtemperatur behandelt. Anschließend wurde die Lösung abdekantiert und die Membran gewaschen (1x DCM, 3x DMF, 3x MeOH, 2x DCM, je 5 min) und getrocknet (Schema II). Beladung 102 µmol/cm².

Ein Membranstück (0.2 µm; aus Beispiel 4) der Größe 5 cm x 3 cm wurde in einer Instrumentenschale 30 min mit einer 2,5 M Lösung von 4,7,10-Trioxa-1,13-tridecandiamin in DCM (v/v) bei 80°C behandelt. Anschließend wurde die Lösung abdekantiert und die Membran gewaschen (1x DCM, 3x DMF, 3x MeOH, 2x DCM, je 5 min) und getrocknet (Schema II). Beladung 152 µmol/cm².

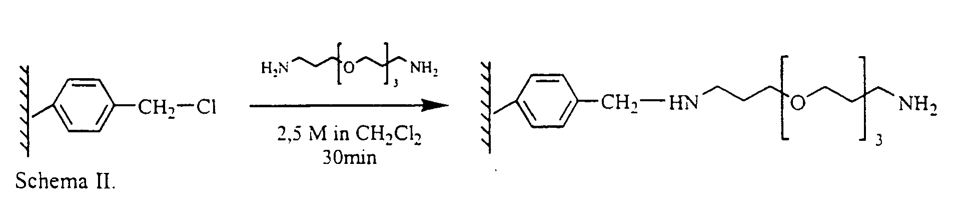

### Beispiel 9: Testung unterschiedlicher Linkersysteme an Hydroxy-PEG-Ester PP - Membranen

Eine Hydroxy-PEG-Ester PP-Membran (0.2 µm, aus Beispiel 1) wurde durch sukzessives Spotten von 1 µl einer 0.6 M NMI-Lösung in NMP und 1 µl einer 0.6 M Lösung von Fmoc-βAla-F in NMP umfunktionalisiert (Amino: Reaktionszeit 2x30 min). Nach vollständiger Abspaltung der Fmoc-Schutzgruppe (Baden der Membran in 20%-iger Piperidin/DMF. 2×15 min) und anschließender Wäsche mit DMF (2x), Methanol (2x) und DCM (2x) und Trocknung an der Luft wurden die folgenden aktivierten Linkermoleküle auf die freien, mit Bromphenolblau angefärbten, Aminofunktionen gespottet:
A) 2 µl 0.6 M Rink-Linker (p-[(R.S)-µ[1-(9H-Fluoren-9-yl)-methoxyformamido]2,4-dimethoxybenzyl]-phenoxyessigsäure) in NMP (5 min voraktiviert mit 1 Eq. TBTU und 2 Eq. DIEA)
B) 2 µl 0.6 M Imidazollinker in NMP (5 min voraktiviert mit 1 Eq. TBTU und 1 Eq. DIEA)
C) 2 µl 0.6 M Aminoethyl-Photolinker 4-[2-Methoxy-4-(1-Fmoc-aminoethyl)-5-nitrophenoxy]-butansäure) in NMP (5 min voraktiviert mit 1 Eq. TBTU und 2 Eq. DIEA)
D) 2 µl 0.6 M Hydroxyethyl-Photolinker 4-[2-Methoxy-4-(hydroxyethyl)-5-nitrophenoxy]-butansäure) in NMP (5 min voraktiviert mit 1 Eq. TBTU und 1 Eq. DIEA)

Die Reaktionszeit betrug in allen Fällen 2 x 30 min. Nach Spaltung der Fmoc-Gruppen wurde das Modellpeptid (Ac-Leu-Lys-Tyr-βAla) mittels Fmoc-Aminosäure-OPfp-Estern (2 µl, 0.3 M in NMP, 2x15 min) unter Spotsynthesebedingungen aufgebaut. Neben der Synthese an Linkersystemen wurde auch eine Synthese direkt auf der βAla-modifizierten Membran durchgeführt. Im Anschluß an das Spotten der 1. Aminosäure wurde die Membran acetyliert, um die Spotgröße zu definieren (Ac₂O, DIEA, DMF; 1:2:7; 30 min) Nach Abspaltung der letzten Fmoc-Gruppe wurden die Peptide erneut acetyliert. Die Seitenkettenschutzgruppen wurden im Fall des Imidazollinkers, des Photolinkers und der direkten Synthese ohne Linker mittels 5% H₂0 in TFA (2 h) deblockiert. Anschließend wurden die Peptide wie folgt abgespalten:
Rink-Linker: 2 h 95% TFA; 5% H₂0
Imidazollinker: 1 h Phosphatpuffer pH: 7.4
Photolinker (C+D): Abspaltung im trockenen Zustand mittels UV-Bestrahlung: 2 h, 365 nm

Nach Eindampfen der TFA und Aufnahme der adhäsiv gebundenen Peptide in 50% ACN/ H₂0 wurde die Qualität der Produkte mittels HPLC und massenpektrometrischer Analyse kontrolliert. Alle auf den neuen Festphasenträgern zum Einsatz gekommenen Linker machten die Isolierung der Produkte mit hoher HPLC-Reinheit (>85%) möglich.

### Beispiel 10: Vergleichende Synthese von Peptiden auf herkömmlich verwendeter Zellulose und Hydroxy-PEG-Ester PP-Membranen

Die Zellulose- und die Hydroxy-PEG-Ester-PP-Membranen (0.2 µm, aus Beispiel 1) wurden mit Fmoc-βAla-F/NMI in NMP acyliert und nach Abspaltung der Fmoc-Gruppe (20% Piperidin/DMF, 20 min) mit dem photolysierbaren Linker 4-2[-Methoxy-4-(1-Fmoc-aminoethyl)-5-nitrophenoxy]-butansäure) modifiziert. Die folgenden Peptide unterschiedlicher Kettenlänge wurden mittels Fmoc-geschützten Aminosäure-Pentafluorphenylestern (2 µl einer 0.3 M Lösung in NMP, Doppelkupplung 2 x 15 min) unter identischen Bedingungen schrittweise aufgebaut:
Ac-Val-Val-Ser-His-Phe-Asn-Asp-Gly-βAla
Ac-Tyr-Ala-Lys-Arg-Cys-Pro-Val-His-Thr-Met-βAla
Ac-Trp-His-Trp-Leu-Gln-Leu-Lys-Pro-Gly-Gln-Pro-Met-Tyr-βAla

Die N-terminale Aminofunktion wurde acetyliert und die Seitenkettenschutzgruppen 2.5 h mittels 5% H₂O, 5% Phenol, 2.5% TIPS in TFA abgespalten. Nach ausgiebiger Wäsche mit DCM und Methanol wurden die Membranen getrocknet und die Peptide durch Bestrahlung mit UV-Licht (2 h, 365 nm) von der Oberfläche gespalten. Die adhäsiv auf der Membran gebundenen Verbindungen konnten nach dem Ausstanzen und Überführen in Mikrotiterplatten mittels Puffer abgelöst und der HPLC/MS-Analytik zugeführt werden.

In allen Fällen besitzen die an der Hydroxy-PEG-Ester-PP-Membran hergestellten Peptide eine höhere Produktreinheit als die an herkömmlicher Zellulose synthetisierten, wie durch die analytischen Profile des Tests zu belegen ist.

### Beispiel 11: Synthese kleiner organischer Moleküle: 1,3,5-Triazin-Bibliothek

Auf eine aminderivatisierte Carboxyl-PP-Membran (siehe Beispiel 7) wurden 2.3 µl einer 0.5 M Lösung von p-[(R,S)-a-[1-(9H-Fluoren-9-yl)-methoxyformamido]2,4-dimethoxybenzyl]-phenoxyessigsäure (Rink-Linker), TBTU (1 Eq.) und DIEA (2 Eq.; Voraktivierzeit: 5 min) im Abstand von 0.8 bis 1 cm gespottet. Nach 15 min wurde das Spotten wiederholt. Nach weiteren 15 min wurde die Membran zweimal 5 min mit MeOH und zweimal 10 min mit DCM gewaschen. Anschließend wurde die Spot-Prozedur wiederholt. Man erhielt nach Fmoc-Quantifizierung Beladungen von ca. 700 nmol/cm², was ca. > 90% der Funktionalitätsdichte der aminderivatisierten Polypropylen-Membran entsprach.

Die mit dem Rink-Linker derivatisierte Membran (100 cm²; 96 Spots) wurde zweimal für 25 min in 75 ml 25proz. Piperidin-Lösung in DMF gebadet und anschließend jeweils zweimal für 10 min mit je 50 ml DMF, MeOH und DCM gewaschen. Zur Acylierung der freien Aminofunktion wurde eine 0.6 M Lösung der Aminosäure mit einem Equivalent TBTU und zwei Equivalenten DIEA in DMF auf die Membran gespottet (2 µl pro Spot). Nach 15 min wurde die Prozedur wiederholt. Nach beendeter Reaktionszeit wurde die Membran jeweils zweimal 10 min mit je 50 ml DMF, MeOH und DCM gewaschen. Die Entschützung der Aminofunktion erfolgte erneut durch zweimaliges Baden der Membran für 25 min in 75 ml 25proz. Piperidin Lösung in DMF und anschließendes Waschen (je 2x, 10 min mit je 50 ml DMF, MeOH und DCM).

Die Aminosäure-derivatisierte Membran wurde dann mit einer 3 M Lösung von Cyanurchlorid mit 3% DABCO in DCM bei 4 °C übergossen und 15 min bei 4°C und 10 min bei Raumtemperatur geschüttelt. Anschließend wurde die Membran 3x mit 50 ml DCM, 2x mit ACN (50 ml) und 2x mit 50 ml DCM je 10 min gewaschen.

Zur Substitution der Chloratome am festphasengebundenen 1,3,5-Triazinderivat spottete man 2µl einer 60%- igen Lösung von prim. oder sek. Aminen (NHR₂R₃) in NMP auf die vorderivatisierte Membran und ließ im geschlossenen Gefäß 25 min bei Raumtemperatur reagieren. Anschließend wurde die Membran dreimal mit je 75 ml DMF (20 min), zweimal mit je 75 ml MeOH (20 min) und dreimal mit je 75 ml DCM (15 min) gewaschen. Auf die luftgetrocknete Membran wurde in einer Glasschale das prim. oder sek. Amin zur Substitution des noch verbleibenden Chloratoms gespottet (2 µl einer 80%-iger. Lösung in NMP) und die Membran in einer Mikrowelle 3 min erhitzt. Die überschüssigen Amine wurden mit DMF (3x je 75 ml, 20 min), 0.1%-iger wässriger Essigsäure (75 ml, 20 min), MeOH (dreimal Essigsäure (75 ml, 20 min), MeOH (dreimal je 75 ml, 20 min) und DCM (dreimal je 75 ml, 20 min) von der Membran entfernt.

Die Abspaltung der Moleküle erfolgte nach Ausstanzen und Überführen in Mikrotiterplatten mittels 150 µl 90%-iger TFA/DCM pro Spot (35 min, 30°C). Nach Abblasen der TFA im Stickstoff-Strom wurden die Substanzen in entsprechenden Puffersystemen aufgenommen und analysiert.

### Beispiel 12: Synthese kleiner organischer Moleküle: Peptoid (N-Benzyl-N-[butyl-(carbamoylmethylcarbamoyl-methyl)carbamoylmethyl-2-[piperidin-1-yl]-acetamid)

Eine aminderivatisierte Carboxyl-PP-Membran (siehe Beispiel 7; vgl. Beispiel 11) wurde mit 30 ml einer 0.3 M Lösung von Fmoc-Glycin in DMF behandelt (30 min), nachdem die Lösung mit 1 Eq. TBTU und 2 Eq. DIEA voraktiviert wurde. Nach dem Waschen der Membran und Schutzgruppenabspaltung (3 x DMF, je 5 min, 1 x 20% Piperidin/DMF, 20 min. 5 x DMF, je 5 min) wurde die Membran erneut mit 30 ml einer frisch angesetzten Lösung von voraktiviertem Fmoc-Glycin versetzt. Nach 30 min Reaktionszeit und Waschen der Membran mit DMF (3 x 5 min) wurden 4 Proben aus der Membran ausgestanzt (0.23 cm²), um den chemisch erreichten Belegungsgrad der Membran zu bestimmen. Dazu wurden die Proben einzeln mit genau 1.00 ml einer 20%-igen Lösung von Piperidin in DMF versetzt und die UV-Absorbtion bei 301 nm gemessen. Es wurde ein Belegungsgrad von 0.45 µmol/cm² erreicht (entspricht 10% der gravimetrisch bestimmten Beladung).

Die mit zwei Glycinspacern versehene Membran wurde dann wie in Beispiel 9 beschrieben mit Rink-Linkersystem modifiziert und nichtumgesetzte Aminofunktionen acetyliert (Ac₂O, DIEA, DCM; 1:2:7). Die nach Abspaltung der Fmoc-Schutzgruppe, Wäsche und Trocknung freigesetzte Aminofunktion wurde durch Aufpipettieren von 0.2 µl einer 0.3 M Fmoc-Glycin-Pentafluorphenylester-Lösung in NMP (3 x 15 min) amidiert. Die Membran wurde wie oben beschrieben gewaschen und die Fmoc-Gruppe abgespalten.

Die Aminofunktion wurde dann durch Pipettierung von 0.2 µl einer 1 M Lösung von Bromessigsäure-2-4 Dinitrophenylester in NMP (3 x 15 min) acyliert. Nach Wäsche mittels DMF (3 x, 5 min) und Methanol (3 x. 5min) und anschließender Trocknung an der Luft wurde durch Spotten von 0.2 µl n-Butylamin (5 M in NMP, 3x 15min) das Brom nukleophil substituiert. Nach Wäsche (3 x 5 min DMF, 3 x 5 min Methanol) und Trocknung an der Luft wurden zwei weitere Synthesezyklen durchgeführt, die sich durch die Natur der verwendeten Amine unterschieden (Zyklus 2: n-Butylamin; Zyklus 4: Piperidin).

Die ausgestanzten Spots wurden wie in Beispiel 9 beschrieben mittels TFA gespalten und die Produkte isoliert und analysiert. Alle untersuchten Produkte zeigten die erwartete Identität bei einer HPLC-Reinheit von >70%.

### Beispiel 13: Synthese von oligomeren Hybriden aus Peptiden und Peptoiden (Peptomere)

Auf eine aminderivatisierte Carboxyl-PP-Membran (siehe Beispiel 7) wurden 2.5 µl einer 0.6 M Lösung von 4-(Methoxy-4-(2-Fmoc-aminoethyl)-5-nitrophenoxyl)-butansäure (photo-labiler Amid-Linker), TBTU (1 Eq.) und DIEA (2 Eq.; Voraktivierzeit: 5 min) im Abstand von 1.0 bis 1.5 cm gespottet. Nach 15 min wurde das Spotten wiederholt. Nach weiteren 15 min wurde die Membran dreimal 5 min mit DMF, zweimal 5 min mit MeOH und zweimal 10 min mit DCM gewaschen. Anschließend wurde die SPOT-Prozedur wiederholt. Man erhielt nach Fmoc-Quantifizierung Beladungen von ca. 700 nmol/cm², was ca. 90% der Funktionalitätsdichte der aminderivatisierten Polypropylen-Membran entsprach.

Die mit dem photolabilen Amid-Linker nicht umgesetzten Aminofunktionen werden acetyliert (Ac₂O, DIEA, DCM; 1:2:7; 30 min) und anschließend dreimal 5 min in DMF gewaschen. Die Fmoc-Schutzgruppe des Linkers wird durch zweimaliges 25 min. Baden der Membran in 75 ml 25% Piperidin in DMF abgespalten. Anschließend wird die Membran jeweils zweimal 10 min mit je 50 ml DMF, MeOH und DCM gewaschen.

Zur Acylierung der freigesetzten Linker-Aminofunktionen werden Fmoc-Aminosäure-Pentafluorphenylester (0.3 M in NMP; Aminosäure-Spots) oder Bromessigsäure-2,4-dinitrophenylester (1.0 M in NMP; Peptoid-Spots) auf die Membran gespottet (2.5 µl pro Spot). Diese Prozedur wird insgesamt dreimal für jeweils 15 min wiederholt. Nach beendeter Reaktionszeit wird die Membran jeweils zweimal 10 min mit je 50 ml DMF, MeOH und DCM gewaschen. Die Entschützung der Fmoc-Aminofunktionen der Aminosäure-Spots erfolgt durch dreimaliges Spotten für 15 min mit 50%iger Piperidin/NMP- Lösung. Gleichzeitig erfolgt die Substitution der Brom-Funktionen der Peptoid-Spots durch Spotten von ein bis fünf molaren Lösungen primärer und sekundärer Amine in DMF, NMP, DMSO. Dimethylacetamid oder Wasser. Nach beendeter Reaktionszeit wird die Membran jeweils zweimal 10 min mit je 50 ml DMF. MeOH und DCM gewaschen.

Die Acylierung der primären Amin-Funktionen (Aminosäure-Spots) und sekundären Amin-Funktionen (Peptoid-Spots) erfolgt entweder durch Spotten (3 x 15 Minuten) von 0.3 M Lösungen von Fmoc-Aminosäure-Pentafluorpenylestern in NMP (Aminosäure-Spots), 0,3 M Fmoc-Aminosäre-Anhydride in NMP (Peptoid-Spots) oder einer 1,0 M Lösung Bromessigsäure-2,4-Dinitrophenylester in NMP (Aminosäure- und Peptoid-Spots). Nach beendeter Reaktionszeit wird die Membran jeweils zweimal 10 min mit je 50 ml DMF, MeOH und DCM gewaschen. Anschließend wird wie oben beschrieben die Synthese fortgesetzt.

Die Fmoc-Aminosäure-Anhydride werden aus den Fmoc-Aminosäuren generiert (0.6 M Fmoc-Aminosäure in NMP: Zusatz von 0.5 Eq. DIC; Voraktivierung: 30 min).

Nach Abschluß der Synthese werden die Seitenkettenschutzgruppen abgespalten (5% H₂O, 5% Phenol, 2.5% TIPS in TFA). Nach ausgiebiger Wäsche mit DCM und Methanol werden die Membranen getrocknet und die Peptide durch Bestrahlung mit UV-Licht (365 nm, 7 mW/cm², 90 min) von der Membran abgespalten.
Die adhäsiv gebundenen Verbindungen konnten nach dem Ausstanzen und Überführen in Mikrotiterplatten mittels Puffer abgelöst und der HPLC/MS-Analytik zugeführt werden. Die verschiedenen Peptomere zeigten vergleichbare hohe Produktqualitäten wobei der jeweils größte Peak der Zielmasse des gewünschten Produktes entsprach.

### Beispiel 14: Untersuchungen zur Biokompatibilität: Antikörperbindungsstudie auf Polymermembranen

Die Epitope Ac-Leu-Pro-Asn-Met-Leu-Arg-Asp-Leu-Arg-Asp-Ala-Phe-Ser-Arg-Val-βAla (mAk CB/RS/13-Epitop); Ac-Ile-Phe-Ile-Asn-Tyr-Ile-Glu-Ala-Tyr-Met-Thr-Met-Lys-Ile-Arg-βAla (mAk CB/RS/11-Epitop); Ac-Val-Val-Ser-His-Phe-Asn-Asp-βAla (mAk Tab2-Epitop) und die unspezifische Testsequenz Ac-His-Val-Asn-Ser-Leu-Gly-Glu-Asn-Leu-Lys-Thr-Leu-Arg-Leu-Arg-βAla wurden jeweils parallel auf herkömmlichen Cellulose-und Polypropylenmembranen unter den Bedingungen der Spot-Synthese (siehe Beispiel 10) synthetisiert.
Nach Abspaltung der Seitenkettenschutzgruppen (2.5 h; 94% TFA, 3% TIPS, 2% H₂0, 1% Phenol) und Wäsche mit DCM (3 x), DMF (4 x) und Methanol (2 x) wurde die methanolfeuchte Membran 3 x 10 min mit Tris Buffered Saline (T-TBS) pH 8.0 gewaschen. Im Anschluß wurde die Membran mit Blockierungspuffer (Boehringer Mannheim, Mannheim, Germany) über 2 h inkubiert und dann 3 x 10 min mit T-TBS-Puffer (pH 8.0) gewaschen. Die so behandelte Membran wurde dann über 1 h mit Peroxidase- (POD) markiertem polyklonalen anti-Maus-IgG Antikörper inkubiert (0.1 µg/ml Blockierungspuffer). Die Antikörperlösung wurde verworfen und die Membran 3 x 10 min mit T-TBS-Puffer (TBS/Tween^{®}20 pH 8.0) gewaschen. Abschließend wurde die Polymermembran mit einem Chemolumineszenz Substrat (Boehringer Mannheim) geschwenkt und Enzymaktivität mit dem Lumi-Imager (Boehringer Mannheim) aufgezeichnet.

| | |
|---|---|
| λ | Lambda (Wellenlänge) |
| AA | Acrylsäure |
| Ac | Acetyl |
| Ac₂O | Essigsäureanhydrid |
| ACN | Acetonitril |
| ATR | Attenuated Total Reflection |
| BP | Benzophenon |
| Chem. Rev. | Chemical Reviews |
| d | Durchmesser |
| DABCO | Diazabicycloundecan |
| DCM | Dichlormethan |
| DIC | Diisopropylcarbodiimid |
| DIEA | Diisopropylethylamin |
| DMF | N,N'-Dimethylformamid |
| DMSO | Dimethylsufoxid |
| DNA | Desoxyribonukleinsäure |
| dₚ | Porendurchmesser |
| ELISA | enzyme linked immunosorbent assay |
| Eq. | Äquivalent |
| Fmoc | 9-Fluorenylmethyloxycarbonyl- |
| Fmoc-βAla-F | N-(9-Fluorenylmethyloxycarbonyl)-β-Alaninfluorid |
| FTIR | Fourier Transform Infrarot Spektroskopie |
| h | Stunden |
| HMBA | 4-Hydroxymethylbenzoesäure |
| HPLC | High Performance Liquid Chromatography |
| i.w. | im wesentlichen |
| J. Am. Chem. Soc. | Journal of the American Chemical Society |
| Leu | Leucin |
| Lys | Lysin |
| M | Mol pro Liter |
| MeOH | Methanol |
| mM | millimolar |
| mm | Millimeter |
| MS | Massenspektrometrie |
| NMI | N-Methylimidazol |
| NMP | N-Methylpyrrolidon |
| OPfp | Pentafluorphenylester |
| PE | Polyethylen |
| PEG | Polyethylenglykol |
| pH | negativer dekadischer Logarithmus der Wasserstoffionenkonzentration |
| PP | Polypropylen |
| prim. | primär |
| proz. | prozentig |
| REM | Rasterelektronenmikroskop |
| RNA | Ribonukleinsäure |
| sek. | sekundär |
| βAla | beta-Alanin |
| TBTU | 2-(1H-Benzotriazol-1-yl)-1,1,3,3-tetramethyluronium tetrafluoroborat |
| Tetrahedron Lett. | Tetrahedron Letters |
| TFA | Trifluoressigsäure |
| TIPS | Triisopropylsilan |
| Tyr | Tyrosin |
| opm | Umdrehungen pro Minute |
| UV | Ultraviolett |
| v/v | volume by volume |

## Patentansprüche

1. Verfahren zur Herstellung eines kontinuierlichen polymeren Festphasenträgers, umfassend eine Trägermatrix mindestens eines polymeren Materials sowie kovalent an die Trägermatrix gebundene Pfropfcopolymerketten, welche mit organischen Verbindungen reagible Reaktivgruppen aufweisen, wobei die Pfropfcopolymerketten räumlich definierte Reaktionsorte ausbilden, mit den Schritten
(a) Beschichten einer Oberfläche der Trägermatrix mit einem Photoinitiator, der geeignet ist, nach UV-Anregung Radikale durch Wasserstoffabstraktion an der Oberfläche der Trägermatrix zu erzeugen, wobei vor der Beschichtung keine Vorbehandlung der Oberfläche mit einem Photoinitiator und mindestens einem Monomer erfolgt,
(b) Belichten der beschichteten Trägermatrix mit UV-Strahlung in Gegenwart mindestens eines ungesättigten funktionellen Monomers, umfassend eine Reaktivgruppe als auch eine polymerisationsfähige Gruppe, wobei die über die polymerisationsfähige Gruppe kovalent an die Trägermatrix gebundenen Pfropfcopolymerketten entstehen, und wobei die Belichtung in Abwesenheit eines vernetzenden Monomers durchgeführt wird, und
(c) Extraktion nicht umgesetzter funktioneller Monomere, des Photoinitiators sowie löslicher Folgeprodukte.

2. Verfahren nach Anspruch 1, **dadurch gekennzeichnet, dass** als Photoinitiator Benzophenon oder ein Benzophenonderivat oder ein mit Benzophenon strukturell verwandtes Keton verwendet wird.

3. Verfahren nach Anspruch 1 oder 2, **dadurch gekennzeichnet, dass** das mindestens eine funktionelle Monomer eine Spacergruppe aufweist, an der sowohl die Reaktivgruppe als auch die polymerisationsfähige Gruppe kovalent, insbesondere über eine Amid-, Ester-, Ether-, Disulfid- oder CC-Gruppe, gebunden ist.

4. Verfahren nach Anspruch 3, **dadurch gekennzeichnet, dass** die Spacergruppe ein Oligoethylenglycol oder ein Polyethylenglycol ist.

5. Verfahren nach einem der vorhergehenden Ansprüche, **dadurch gekennzeichnet, dass** die polymerisationsfähige Gruppe eine reaktionsfähige Mehrfachbindung umfasst.

6. Verfahren nach Anspruch 5, **dadurch gekennzeichnet, dass** die polymerisationsfähige Gruppe eine Acrylat-, Vinyl- oder Allylgruppe ist.

7. Verfahren nach einem der vorhergehenden Ansprüche, **dadurch gekennzeichnet, dass** die Reaktivgruppe eine Hydroxyl-, Amino-, Carboxyl-, Epoxy-, Mecapto- oder Halogengruppe ist.

8. Verfahren nach einem der vorhergehenden Ansprüche, **dadurch gekennzeichnet, dass** das mindestens eine funktionelle Monomer gewählt ist aus der Gruppe, umfassend Polyethylenglycolmethacrylat, Amino(polyethylenglycol)methacrylat, Amino(polyethylenglycol)methacrylamid, Acrylsäure, 2-Hydroxypropylmethacrylamid und Vinylbenzylchlorid.

9. Verfahren nach einem der vorhergehenden Ansprüche, **dadurch gekennzeichnet, dass** die polymere Trägermatrix mindestens ein auf einer polyolefinischen Basisstruktur beruhendes Polymer umfasst, insbesondere Polypropylen, Polyethylen, Polyvinylidenfluorid, Polytetrafluorethylen oder vernetztes Polystyren.

10. Verfahren nach einem der vorhergehenden Ansprüche, **dadurch gekennzeichnet, dass** nach der Pfropfcopolymerisation die Reaktivgruppe chemisch umfunktionalisiert wird.

11. Verfahren nach einem der vorhergehenden Ansprüche, **dadurch gekennzeichnet, dass** eine Bindung zwischen Reaktivgruppe und Spacergruppe oder zwischen Spacergruppe und polymersiationsfähiger Gruppe hydrolytisch und/oder oxidativ/reduktiv spaltbar ist.

12. Verwendung eines nach einem der Ansprüche 1 bis 11 hergestellten kontinuierlichen polymeren Festphasenträgers zur parallelen und kombinatorischen Synthese von Verbindungen, wobei die Synthese ein sequentielles Spotten aktivierter Synthesebausteine oder Reagenzien auf die verschiedenen Reaktionsorte des Festphasenträgers umfasst, so dass eine Bibliothek von an dem Festphasenträger über die funktionellen Gruppen der Pfropfcopolymerkette gebundenen Verbindungen erhalten wird und jeder Reaktionsort des Festphasenträgers die Zusammensetzung der synthetisierten Verbindung bestimmt.

13. Verwendung nach Anspruch 12, **dadurch gekennzeichnet, dass** die synthetisierten Verbindungen nach der Synthese von der Trägermatrix abgespalten werden oder gebunden bleiben.

14. Verwendung nach Ansprüche 12 oder 13, **dadurch gekennzeichnet, dass** eine Mehrzahl von identisch oder unterschiedlich funktionalisierten Festphasenträgern in Form eines Arrays oder eines Stapels für die parallele und kombinatorische Synthese von multipel oder graduell abspaltbaren Verbindungen eingesetzt wird.

15. Verwendung nach einem der Ansprüche 12 bis 14, **dadurch gekennzeichnet, dass** die aus aktivierten Synthesebausteinen synthetisierten Verbindungen Peptide, Proteine, Peptoide, Peptomere, Oligocarbamate, Oligoharnstoffe, Azatide, Ketide, Peptid-Sulfonamide, vinyloge Sulfonylpeptide, Desoxynukleinsäuren oder Peptid-Nukleinsäuren sind.

16. Verwendung nach einem der Ansprüche 12 bis 14, **dadurch gekennzeichnet, dass** die Synthese der Verbindungen ein sequentielles Spotten von Reagenzien auf eine an den Festphasenträger gebundene Kernstruktur umfasst, wobei variable Gruppen eingeführt werden.

17. Verwendung nach Anspruch 16, **dadurch gekennzeichnet, dass** die Synthese der Verbindungen einen Heterozyklisierungsschritt und/oder Mehrkomponentenreaktionen umfasst.

18. Verwendung nach Anspruch 16 oder 17, **dadurch gekennzeichnet, dass** die synthetisierten Verbindungen kleine organische Verbindungen sind, insbesondere Benzodiazepine, Triazine, Triazole, Hydantoine, Cubane, Xanthine, Pyrollidine, β-Lactame, Thiazolidone, Isoquinoline oder Diketopiperazine sind.

19. Verwendung eines nach einem der Ansprüche 12 bis 18 hergestellten kontinuierlichen polymeren Festphasenträgers, der die an diesem gebundenen Verbindungen trägt, zur Identifizierung von Liganden, wobei die Identifizierung eine Inkubation des Festphasenträgers mit dem zu identifizierenden Ligand, eine Entfernung überschüssiger Liganden durch Waschen und Detektion der an den Verbindungen des Festphasenträgers gebundenen Liganden durch ein geeignetes Verfahren umfasst.

20. Verwendung nach Anspruch 19, **dadurch gekennzeichnet, dass** das Verfahren zur Detektion ein'immunologischer Nachweis, ein Nachweis radioaktiv markierter Liganden, ein Fluoreszenz- oder Chemolumineszenz-Nachweis oder ein biosensorischer Nachweis ist.

21. Verwendung eines nach einem der Ansprüche 12 bis 18 hergestellten kontinuierlichen polymeren Festphasenträgers, der die an diesem gebundenen Verbindungen trägt, zur Untersuchung einer enzymatischen Aktivität von Enzymen, wobei die Untersuchung eine Inkubation des Festphasenträgers mit dem Enzym und Detektion der enzymatische Aktivität der an den Verbindungen gebundenen Enzyme umfasst.

## Claims

1. Method for producing a continuous polymeric solid phase supporting material, comprising a supporting matrix of at least one polymeric material and graft copolymer chains which are covalently bound to the supporting matrix and have reactive groups being able to react with organic compounds, the graft copolymer chains forming spatially defined reaction sites, comprising the steps of
(a) coating a surface of the supporting matrix with a photoinitiator which is suitable, after UV excitation, of generating radicals at the surface of the supporting matrix by hydrogen abstraction, no pre-treatment of the surface with a photoinitiator and at least one monomer taking place before the coating,
(b) exposing the coated supporting matrix to UV radiation in the presence of at least one unsaturated functional monomer, comprising a reactive group and also a group capable of polymerisation, wherein the graft polymer chains which are covalently bound to the supporting matrix via the group capable of polymerisation are produced, and wherein the exposure is carried out in the absence of a cross-linking monomer, and
(c) extraction of non-reacted functional monomers, of the photoinitiator and soluble secondary products.

2. Method according to claim 1, **characterised in that** benzophenone or a benzophenone derivative or a ketone structurally related to benzophenone is used as photoinitiator.

3. Method according to claim 1 or 2, **characterised in that** the at least one functional monomer has a spacer group, to which both the reactive group and also the group which is capable of polymerisation is covalently bound, in particular via an amide, ester, ether, a disulphide or a CC group.

4. Method according to claim 3, **characterised in that** the spacer group is an oligoethylene glycol or a polyethylene glycol.

5. Method according to any one of the preceding claims, **characterised in that** the group which is capable of polymerisation comprises a reactive multiple bond.

6. Method according to claim 5, **characterised in that** the group which is capable of polymerisation is an acrylate, vinyl or allyl group.

7. Method according to any one of the preceding claims, **characterised in that** the reactive group is a hydroxyl, amino, carboxyl, epoxy, mecapto or halogen group.

8. Method according to any one of the preceding claims, **characterised in that** the at least one functional monomer is selected from the group comprising polyethylene glycol methacrylate, amino(polyethylene glycol)methacrylate, amino(polyethylene glycol)methacrylamide, acrylic acid, 2-hydroxypropylmethacrylamide and vinyl benzyl chloride.

9. Method according to any one of the preceding claims, **characterised in that** the polymeric supporting matrix comprises at least one polymer based on a polyolefinic base structure, in particular polypropylene, polyethylene, polyvinylidene fluoride, polytetrafluoroethylene or cross-linked polystyrene.

10. Method according to any one of the preceding claims, **characterised in that** the reactive group is chemically refunctionalised after the graft polymerisation.

11. Method according to any one of the preceding claims, **characterised in that** a bond between the reactive group and spacer group or between the spacer group and group capable of polymerisation can be cleaved hydrolytically and/or by oxidation/reduction.

12. Use of a continuous polymeric solid phase supporting material produced according to any one of claims 1 to 11 for parallel and combinatory synthesis of compounds, the synthesis comprising a sequential spotting of activated synthesis building blocks or reagents on the different reaction sites of the solid phase supporting material, so that a library of compounds bound to the solid phase supporting material via the functional groups of the graft copolymer chain is obtained and each reaction site of the solid phase carrier determines the composition of the synthesised compound.

13. Use according to claim 12, **characterised in that** the synthesised compounds, after synthesis, are cleaved from the supporting material matrix or remain bound.

14. Use according to claims 12 or 13, **characterised in that** a plurality of identically or differently functionalised solid phase carrier materials are used in the form of an array or a stack for the parallel and combinatory synthesis of multiply or gradually cleavable compounds.

15. Use according to any one of claims 12 to 14, **characterised in that** the compounds synthesised from activated synthesis building blocks are peptides, proteins, peptoids, peptomers, oligocarbamates, oligoureas, azatides, ketides, peptide sulphonamides, vinylogenic sulphonyl peptides, deoxynucleic acids or peptide nucleic acids.

16. Use according to any one of claims 12 to 14, **characterised in that** the synthesis of the compounds comprises a sequential spotting of reagents on a core structure bound to the solid phase supporting material, variable groups being introduced.

17. Use according to claim 16, **characterised in that** the synthesis of the compounds comprises a heterocycling step and/or multi-component reactions.

18. Use according to claim 16 or 17, **characterised in that** the synthesised compounds are small organic compounds, in particular benzodiazepines, triazines, traizoles, hydantoins, cubans, xanthines, pyrrolidines, β-lactams, thiazolidones, isoquinolines or diketopiperazines.

19. Use of a continuous polymeric solid phase supporting material produced according to any one of claims 12 to 18, which carries the compounds bound thereto, for identifying ligands, wherein the identification comprises incubation of the solid phase supporting material with the ligand to be identified, removal of excess ligands by washing and detection of the ligands bound to the compounds of the solid phase supporting material by a suitable method.

20. Use according to claim 19, **characterised in that** the method for detection is immunological detection, detection of radioactively marked ligands, fluorescence or chemoluminescence detection or biosensory detection.

21. Use of a continuous polymeric solid phase supporting material produced according to any one of claims 12 to 18, which carries the compounds bound thereto, for investigating an enzymatic activity of enzymes, the investigation comprising incubation of the solid phase supporting material with the enzyme and detection of the enzymatic activity of the enzymes bound to the compounds.

## Revendications

1. Procédé de préparation d'un support en phase solide polymère continu, comprenant une matrice support d'au moins un matériau polymère et des chaînes de copolymère greffé, liées par covalence à la matrice support, qui présentent des groupes réactifs pouvant réagir avec des composés organiques, les chaînes de copolymère greffé formant des sites de réaction spatialement définis, comprenant les étapes de :
(a) revêtement d'une surface de la matrice support avec un photoamorceur qui convient pour produire, après activation par des UV, des radicaux par abstraction d'hydrogène sur la surface de la matrice support, aucun prétraitement de la surface avec un photoamorceur et au moins un monomère ne s'effectuant avant le revêtement,
(b) exposition de la matrice support revêtue à un rayonnement UV en présence d'au moins un monomère fonctionnel insaturé, comprenant un groupe réactif et un groupe polymérisable, grâce à quoi il se forme des chaînes de copolymère greffé liées par covalence à la matrice support par l'intermédiaire du groupe polymérisable, et l'exposition s'effectuant en l'absence de monomère réticulant,
(c) extraction des monomères fonctionnels n'ayant pas réagi, du photoamorceur et des produits secondaires solubles.

2. Procédé selon la revendication 1, **caractérisé en ce que** l'on utilise comme photoamorceur de la benzophénone ou un dérivé de benzophénone ou une cétone structuralement apparentée à la benzophénone.

3. Procédé selon la revendication 1 ou 2, **caractérisé en ce qu'**au moins un monomère fonctionnel présente un groupe espaceur auquel sont liés par covalence aussi bien le groupe réactif que le groupe polymérisable, en particulier par l'intermédiaire d'un groupe amide, ester, éther, disulfure ou CC.

4. Procédé selon la revendication 3, **caractérisé en ce que** le groupe espaceur est un oligoéthylèneglycol ou un polyéthylèneglycol.

5. Procédé selon l'une des revendications précédentes, **caractérisé en ce que** le groupe polymérisable comprend une liaison multiple réactive.

6. Procédé selon la revendication 5, **caractérisé en ce que** le groupe polymérisable est un groupe acrylate, vinyle ou allyle.

7. Procédé selon l'une des revendications précédentes, **caractérisé en ce que** le groupe réactif est un groupe hydroxyle, amino, carboxyle, époxy, mercapto ou halogéno.

8. Procédé selon l'une des revendications précédentes, **caractérisé en ce qu'**au moins un monomère fonctionnel est choisi dans le groupe comprenant un méthacrylate de polyéthylèneglycol, un méthacrylate d'amino(polyéthylèneglycol), un amino(polyéthylèneglycol)méthacrylamide, l'acide acrylique, le 2-hydroxypropylméthacrylamide et le chlorure de vinylbenzyle.

9. Procédé selon l'une des revendications précédentes, **caractérisé en ce que** la matrice support polymère comprend au moins un polymère à base d'une structure de base polyoléfinique, en particulier du polypropylène, du polyéthylène, du poly(fluorure de vinylidène), de polytétrafluoroéthylène ou du polystyrène réticulé.

10. Procédé selon l'une des revendications précédentes, **caractérisé en ce que**, après la copolymérisation par greffage, on soumet le groupe réactif à une transfonctionnalisation chimique.

11. Procédé selon l'une des revendications précédentes, **caractérisé en ce qu'**une liaison entre le groupe réactif et le groupe espaceur ou entre le groupe espaceur et le groupe polymérisable peut être coupée par hydrolyse et/ou par oxydation/réduction.

12. Utilisation d'un support en phase solide polymère continu préparé selon l'une des revendications 1 à 11 pour la synthèse parallèle et combinatoire de composés, la synthèse comprenant une série de dépôts d'éléments constitutifs de synthèse activés ou de réactifs sur les différents sites de réaction du support en phase solide, de sorte que l'on obtient une banque de composés liés au support en phase solide par l'intermédiaire des groupes fonctionnels de la chaîne de copolymère greffé et que chaque site de réaction du support en phase solide détermine la composition du composé synthétisé.

13. Utilisation selon la revendication 12, **caractérisée en ce que** les composés synthétisés sont séparés de la matrice support après la synthèse ou restent liés.

14. Utilisation selon la revendication 12 ou 13, **caractérisée en ce que** l'on utilise une pluralité de supports en phase solide fonctionnalisés de manière identique ou différente sous forme d'une puce ou d'un empilement pour la synthèse parallèle et combinatoire de composés pouvant être séparés sous forme multiple ou progressive.

15. Utilisation selon l'une des revendications 12 à 14, **caractérisée en ce que** les composés synthétisés à partir d'éléments constitutifs de synthèse activés sont des peptides, des protéines, des peptoïdes, des peptomères, des oligocarbamates, des oligourées, des azatides, des kétides, des peptide-sulfonamides, des sulfonylpeptides vinylogues, des acides désoxynucléiques ou des acides nucléiques peptidiques.

16. Utilisation selon l'une des revendications 12 à 14, **caractérisée en ce que** la synthèse des composés comprend une série de dépôts de réactifs sur une structure de noyau liée au support en phase solide, grâce à quoi des groupes variables sont introduits.

17. Utilisation selon la revendication 16, **caractérisée en ce que** la synthèse des composés comprend une étape d'hétérocyclisation et/ou des réactions à plusieurs constituants.

18. Utilisation selon la revendication 16 ou 17, **caractérisée en ce que** les composés synthétisés sont de petits composés organiques, en particulier des benzodiazépines, des triazines, des triazoles, des hydantoïnes, des cubanes, des xanthines, des pyrrolidines, des β-lactames, des thiazolidones, des isoquinoléines ou des dicétopipérazines.

19. Utilisation d'un support en phase solide polymère continu préparé selon l'une des revendications 12 à 18, qui porte les composés qui lui sont liés, pour l'identification de ligands, l'identification comprenant l'incubation du support en phase solide avec le ligand à identifier, la séparation du ligand en excès par lavage et la détection du ligand lié aux composés du support en phase solide par un procédé approprié.

20. Utilisation selon la revendication 19, **caractérisée en ce que** le procédé de détection est une détection immunologique, une détection de ligands marqués par une substance radioactive, une détection par fluorescence ou chimioluminescence ou une détection avec un biocapteur.

21. Utilisation d'un support en phase solide polymère continu préparé selon l'une des revendications 12 à 18, qui porte les composés qui lui sont liés, pour l'analyse de l'activité enzymatique d'enzymes, l'analyse comprenant l'incubation du support en phase solide avec l'enzyme et la détection de l'activité enzymatique des enzymes liées aux composés.
